# EUROPEAN PATENT SPECIFICATION

(11) **EP 2 395 950 B1**
(45) Date of publication and mention of the grant of the patent: **16.12.2015**
(21) Application number: 10704173.3
(22) Date of filing: 21.01.2010
(51) Int. Cl.: A61F 5/01

(54) **SHOULDER STABILISING DEVICE**
SCHULTERSTABILISIERUNGSVORRICHTUNG
DISPOSITIF DE STABILISATION D'ÉPAULE

(30) Priority: 21.01.2009 GB 0900939
(43) Date of publication of application: 21.12.2011
(73) Proprietor: NHS South West Essex, Basildon, Essex SS14 3HG (GB)
(72) Inventor: MATTHIAS, Usharani, Stanford-Ly-Hope Essex SS17 8BF (GB)
(74) Representative: Miller, David James
(86) International application number: PCT/GB2010/050090
(87) International publication number: WO 2010/084352

(56) References cited:
- WO-A1-00/25708
- WO-A1-2009/017442
- US-A- 3 277 889
- US-A1- 2005 119 596
- US-A1- 2007 106 187
- US-A1- 2008 039 764

## Description

The invention relates to a shoulder stabilising device which enables the correct position of the humeral head to be maintained within the glenohumeral joint. The disclosure also relates to shoulder stabilising kits and to a method of stabilising the shoulder. The invention is defined in claim 1.

The glenohumeral (GH) joint is a ball and socket joint with three rotational and three translational degrees of freedom. The other joints related to the GH joint are the sternoclavicular (SC) joint, the acromioclavicular (AC) joint and the scapulothorasic joints which form the shoulder complex. The GH joint has a capsule and several associated ligaments and bursae. The articulation is composed of the large head of the humerus and smaller glenoid fossa. The GH joint has sacrificed articular congruency to serve the mobility needs of the arm and is subsequently susceptible to degenerative changes, instability and derangement. The supraspinatus, infraspinatus, teres minor and subscapularis muscles compose the rotator cuff. These muscles work together to lift and rotate the shoulder. The rotator cuff muscles provide dynamic stabilization to the humeral head on the glenoid fossa. There must be a balance between mobility and stability to retain proper function, and it is this balance that embodies the biomechanics of the shoulder complex.

Impingement is one of the most common causes of pain in the shoulder. The pain results from pressure on the rotator cuff from part of the shoulder blade (scapula) as the arm is lifted. The rotator cuff muscles, especially the supraspinatus tendon, are compressed further when the humerus is placed in the forward-flexed and internally rotated position forcing the greater tuberosity of the humerus into the under surface of the acromion and coracoacromial arch.

Another common form of shoulder injury is a torn rotator cuff muscle. It is estimated that up to 5% of the population have either a full or a partial thickness rotator cuff tear and that 40% of these will seek medical attention. Studies have shown that the incidence of such injuries increases with age as the muscle weakens. As the population ages during the coming decades, such injuries will therefore become even more prevalent. Treatment for full thickness tears requires surgery followed by post-operative physiotherapy-based rehabilitation, while partial thickness tears may be treatable by physiotherapy alone. Stabilising the shoulder joint during such episodes of rehabilitation has been shown to significantly improve the extent and rate of recovery and is thus a desirable addition to physiotherapy.

Known shoulder stablilisers are frequently intended to promote healing of serious injuries such as dislocations, subluxations or fractures. Braces for this purpose can be elaborate and may comprise a plurality of straps that pass around the body and the affected shoulder or arm to stabilise and restrict movement of that shoulder. This may also be combined with a portion which the affected arm rests on to further reduce movement (as disclosed in US 2005/0010147). Such systems can be complex to use and may require the assistance of another, potentially medically skilled person to be fitted. They may also be uncomfortable and impractical for use in an everyday setting for example in the work place.

Shoulder braces more appropriate for conditions to be treated by the present invention tend to comprise a portion worn over the affected shoulder and/or part of the torso. As in devices described hereinbefore, they also incorporate a system of straps which may pass around the body and require the assistance of another when being fitted. They are therefore not straightforward to use. Another disadvantage of these braces is that they also limit movement of the affected arm to a greater degree than is necessary in the current context. Examples of shoulder stabilisers are disclosed in US 5,628,725 and WO 2008/105671.

Document D1 (US 2007/106187) provides a shoulder brace with a pneumatic pad for increasing or decreasing pressure against the humeral head of the shoulder.

Document D2 (US 2005/119596) provides a shoulder brace for providing support to the shoulder area.

Document D3 (US 3,277,889) provides a clavicle brace for the effective treatment of fractures of the clavicle or collar bone.

Accordingly, there is a need in the art for a new shoulder stabilising device. This need is addressed by the present invention, which solves one or more of the above-mentioned problems.

According to a first instance of the disclosure there is provided a shoulder stabilising device for maintaining the correct position of the humeral head within the glenohumeral joint of a human subject, comprising pressure application means configured to apply pressure at the anterior margin of the glenohumeral joint.

In an aspect of the invention, there is provided a shoulder stabilising device comprising:
a pressure pad that is capable of being positioned on an anterior margin of a glenohumeral joint of a human subject;
wherein the shoulder stabilising device is configured, in use, to apply a focal pressure, via the pressure pad, to the anterior margin of the glenohumeral joint, without applying direct pressure to the humeral head, whereby said focal pressure causes posterior translation of the humeral head within the glenohumeral joint.

More specifically, the shoulder stabilising device is configured, in use, to apply a focal pressure, via the pressure pad, to the lesser tuberosity/tubercle of the humerus of the subject. Thus, as used herein, references to the "anterior margin" also include references to the lesser tuberosity/tubercle. Accordingly, the shoulder stabilising device does not apply direct pressure to the humeral head.

On a diseased or damaged rotator cuff the integrity of the tension created by the muscle is compromised causing anterior translation of the humeral head. The device of the present invention is placed at the anterior margin of the GH joint space under tension and advantageously causes posterior translation of the humeral head by applying focal pressure to the anterior margin (e.g. the lesser tuberosity/tubercle) without applying direct pressure to the humeral head, thereby relocating the humeral head to the correct position within the GH joint (more specifically, within the glenoid fossa). Without being bound by theory, this relocation reduces the impingement of the subacromial space allowing pain free movement of the GH joint. In the case of a torn rotator cuff muscle, this relocation will serve to stabilise the shoulder joint during the period of rehabilitation.

The invention is further designed to provide stability to a shoulder joint while minimising the complexity of apparatus required to achieve this goal. The invention is intended for everyday use by patients suffering from impingement-related shoulder pain or recovering from a rotator cuff injury rather than athletes wishing to maximise the use of a damaged or weakened shoulder. As the principle function of the invention is to treat simple anterior translation of the humeral head, by providing posterior translation, the device can be substantially less complex in design than known stabilising devices. There are several significant advantages resulting from this simplicity. Firstly, the device is easier to use than known devices because it does not require complex strapping to maintain its correct position. Secondly, the restriction of movement of the glenohumeral joint is kept to a minimum. With any treatment of shoulder instability, a balance must be struck between stabilising the joint and allowing free movement of the affected arm. As the present invention specifically targets one type of joint instability it has the benefit of not overly restricting normal movement of the arm and shoulder. Finally, the device is unobtrusive and comfortable to wear, making it suitable for continued use throughout the day.

References herein to 'pressure pad' relate to a member that is substantially flat and can lie on the anterior surface of the shoulder without being overly conspicuous or causing any significant disruption to the subject's normal daily activities. The pressure pad may be constructed of a material that is comfortable for the subject to wear.

The pressure pad may range in depth from approximately 1 cm to 3 cm. It will be appreciated that the surface area of the pressure pad will be such to cover an area of the shoulder sufficient to effectively apply a focal pressure to the anterior margin.

As used herein, the term "focal pressure" will be understood to mean a pressure that is applied to a focused (i.e. small) area, e.g. a surface area of about 1-100 cm², such as about 1-50 cm², such as about 3-39 cm², e.g. about 3-5, 3-10, 3-15, 3-20, 3-25, 3-30, 3-35 or 3-39 cm². The device may apply a force of about 0.1-100 N, such as about 0.1-50 N, about 0.1-20 N, about 0.1-10 N, e.g. about 0.1, 1, 5, 10, 20, 30, 40, 50, 60, 70, 80, 90 or 100 N.

In certain embodiments, the shoulder stabilising device is configured, in use, to apply a focal pressure by means of the pressure pad to a single position of the anterior margin (e.g. to the lesser tuberosity/tubercle). The advantage of the invention is that it applies pressure only to the required portion of the shoulder (i.e. the anterior margin, e.g. the lesser tuberosity/tubercle) without applying direct pressure to the humeral head, maximising the beneficial effect achieved whilst minimising the size and complexity of the device.

In one embodiment, the pressure pad applies the focal pressure by means of its shape, i.e. it has a surface area of about 1-100 cm², such as about 1-50 cm², such as about 3-39 cm², e.g. about 3-5, 3-10, 3-15, 3-20, 3-25, 3-30, 3-35 or 3-39 cm². In this regard, the surface area of the pressure pad may correspond to the surface area of the lesser tuberosity/tubercle. In addition, or in the alternative, the shape of the pressure pad may correspond to the shape of the lesser tuberosity/tubercle, and may therefore be tear-drop shaped, round, oval or square.

In another embodiment, the pressure pad applies a focal pressure to one or more positions of the anterior margin of the glenohumeral joint by means of one or more protruding members. Thus, the pressure pad may comprise one or more protruding members that are each configured, in use, to apply focal pressure so as to cause posterior translation of the humeral head.

In a further embodiment, the pressure pad comprises a single protruding member that applies a focal pressure to a single position of the anterior margin (e.g. to the lesser tuberosity/tubercle).

The one or more protruding members may each have a surface area of about 1-100 cm², such as about 1-50 cm², such as about 3-39 cm², e.g. about 3-5, 3-10, 3-15, 3-20, 3-25, 3-30, 3-35 or 3-39 cm². In this regard, the surface area of each protruding member may correspond to the surface area of the lesser tuberosity/tubercle.

The one or more protruding members may form part of the surface of the pressure pad. Alternatively, the pressure pad may be constructed of a peripheral portion configured to maintain contact with the anterior shoulder of the subject and a central portion configured to accommodate one or more protruding members.

The protruding members may be resiliently biased away from the pressure pad by e.g. a spring. In this manner, the protruding members are configured, in use, to be resiliently biased against the anterior margin of the human subject.

The one or more protruding members may typically have a semi-spherical profile, with the rounded portion contacting the shoulder of the subject. It will however be appreciated that any profile allowing concentrated and comfortable application of pressure will be suitable. The cross-sectional shape of the one or more protruding members may correspond to the shape of the lesser tuberosity/tubercle, and may therefore be tear-drop shaped, round, oval or square.

The protruding members may be constructed of a soft but resilient material such as rubber or an elastomeric polymer. The extent to which the members protrude from the pressure pad will vary depending on the requirements of the patient but will typically be in the range of 2mm to 30mm, e.g. 2mm to 20mm.

The protruding members may be adjustable in size and/or shape. In addition, or in the alternative, the one or more protruding members may be interchangeable with other protruding members. The interchangeable protruding members can be manufactured in a range of sizes and materials so that changing the protruding members can alter the pressure applied by the device, in order to treat many different treatment requirements.

In one embodiment, the protruding members can be moved to different positions within the pressure pad so as to apply pressure to the required part of the anterior margin. For example, the protruding members can be moved up and down (i.e. vertically) and/or left and right (i.e. horizontally) within the surface of the pressure pad. This embodiment has the advantage that pressure can be applied in precisely the right location on the shoulder, e.g. so that pressure can be applied to the lesser tuberosity/tubercle.

In one embodiment, the shoulder stabilising device additionally comprises a pressure adjustor that is capable of adjusting the pressure applied by the device, in use, to the anterior margin of the glenohumeral joint. This embodiment has the advantage that the device may be used for many different human subjects. Alternatively, a single subject may wish to alter the amount or nature of pressure applied to their shoulder during a course of treatment. The pressure adjustor may be selected from, but not limited to, one or more of a buckle, a screw adjustment, a ratchet mechanism or one or more inflatable members.

In one embodiment, the pressure adjustor is a screw adjustment mechanism. In this embodiment, turning the screw in one direction increases the pressure exerted on the shoulder while turning the screw in the other direction decreases the pressure exerted on the shoulder. This embodiment has the advantage that many different levels of pressure application can be achieved with a single device.

In one embodiment, the screw adjustment additionally comprises one or more graduations. This embodiment provides the advantage that the degree of pressure can be quantified, allowing the subject to keep a record of how much pressure is being applied during the course of treatment.

In an alternative embodiment, the pressure adjustor is a ratchet mechanism. This embodiment shares all the advantages of the screw adjustment embodiment but uses a different system to enable variable application of pressure.

In an alternative embodiment the pressure adjustor comprises one or more inflatable members. The inflatable member(s) can be inflated or deflated to provide greater or lesser pressure on the anterior margin. In a further embodiment, the device additionally comprises an inflation means. Examples of such inflation means include a pump or bulb. In a yet further embodiment, the device additionally comprises an inflation valve. The inflation valve can be used to decrease pressure (by releasing gas to the atmosphere) or to increase pressure (by fitting the inflation means and inflating the inflatable members).

In one embodiment, the shoulder stabilising device additionally comprises one or more retention members. These can be used to secure the pressure pad, in use, on the anterior margin of the glenohumeral joint. The one or more retention members allow the subject to easily move the shoulder stabilising device to the desired location on the shoulder.

The retention members can be connected to each other to provide additional support. The one or more retention members can be removably attached to each other and/or to the pressure pad. For instance, the retention members may additionally comprise a pocket or recess configured to receive the pressure pad or other retention member. In addition, or in the alternative, the retention members may be provided with Velcro®.

In certain embodiments, the retention members are configured to urge the pressure pad towards the anterior margin.

The one or more retention members may be constructed from a flexible material which is comfortable to wear. Suitable materials include for example elastic material (such as rubber), cotton, linen, webbing, polyester, nylon, and the like, or any combination of these and other materials.

In one embodiment, the one or more retention members are selected from the group consisting of a support strap, a support band, a support belt, a support leash and/or a support harness.

The support strap may be an elastic strap which vertically surrounds the shoulder of the subject. The dimensions of the support strap and the material it is made from can be adjusted to the individual subject's requirements. The support strap may be constructed from a thermoplastic elastomer such as Hytrel® or other durable polymer such as Zytel® ST. Another suitable material for the support strap is a silicone elastomer. A portion of the support strap located over the glenohumeral joint can be configured to receive the pressure pad. The support strap can be adjusted to fit the subject by adjustment means, such as a buckle or a Velcro® fitting.

The support harness runs around the body of the subject and helps to ensure that the shoulder stabilising device remains in the correct position.

In one embodiment, at least one of the retention members is a support pad. References herein to 'support pad' relate to a member that is substantially flat and can lie on the surface of the posterior shoulder without being overly conspicuous or causing any significant disruption to the subject's normal daily activities. The support pad may be constructed of a material that is comfortable for the subject to wear.

The support pad may range in depth from approximately 1 cm to 3 cm. It will be appreciated that the surface area of the support pad will be such to cover an area of the shoulder sufficient to effectively maintain the pressure pad in the correct position on the surface of the shoulder. In one embodiment, the support pad has a surface area of about 1-100 cm², such as about 1-50 cm², such as about 3-39 cm², e.g. about 3-5, 3-10, 3-15, 3-20, 3-25, 3-30, 3-35 or 3-39 cm².

The device may additionally comprise a joining portion that connects the pressure pad to the support pad. The joining portion may be configured, in use, to urge the pressure pad towards the anterior margin. In one embodiment, the joining portion may also be configured to urge the pressure pad and the support pad towards each other so as to apply a compressive force to the shoulder.

In one embodiment, the pressure pad and the support pad are articulated with respect to the joining portion. Articulation may be provided by a movable member, for example a hinge. The moveable member connected to the pressure pad may also be spring-loaded with respect to the joining portion in order to urge the pressure pad towards the anterior margin. The moveable member connected to the support pad may also be spring-loaded with respect to the joining portion so as to apply a compressive force to the shoulder.

The size of the joining portion may vary to take into account anatomical differences between subjects. In one embodiment, the joining portion may comprise adjusting means so that its size can be adjusted.

The joining portion may be roughly semi-circular in shape and configured to partially surround the shoulder of the subject, thereby forming an arch around the joint. The joining portion may run either above or below the shoulder. In one embodiment, the joining portion runs over the top of the shoulder, optionally resting directly on the surface of the shoulder. The joining portion is therefore immobilised on top of the shoulder, thereby maintaining the correct position of the shoulder stabilising device.

The joining portion may typically have a substantially flat cross-section. As a result, the shoulder stabilising device will cause minimum inconvenience to the subject wearing it and will allow it to be worn easily under clothing.

In one embodiment, the shoulder stabilising device (e.g. the pressure pad, support pad and joining portion) may be moulded from a single piece of rigid material, e.g. a durable plastic such as polyoxymethylene (Delrin®), acetal-based plastics and glass-filled acetal-based plastics, polysulfone or polycarbonate or a lightweight metal such as titanium or aluminium (or alloys of either metal). The rigidity of the material can advantageously provide the pressure required to cause posterior translation of the humeral head (and thereby to realign the GH joint) without the need for other apparatus or strapping.

In one embodiment, the shoulder stabilising device is covered in a cushioning material. This embodiment provides the advantage of maximising comfort to the subject wearing the shoulder stabilising device. Examples of cushioning materials include textiles, fabrics, silicone rubber, natural rubber, neoprene, polyurethane or other foam materials, ethylene vinyl acetate, polybutadiene and various thermoplastic elastomers.

In one embodiment, the shoulder stabilising device (e.g. the pressure pad, support pad and joining portion) is coated in or made of a material which increases frictional resistance with the skin of the human subject. Its surface may be coated in or made of a soft adhesive or 'tacky' material which easily adheres to the surface of the subject's skin. This ensures that the correct position of the device is maintained.

According to a second instance of the disclosure, there is provided a shoulder brace comprising one or two shoulder stabilising devices as hereinbefore defined. In one instance the shoulder brace comprises two shoulder stabilising devices (one for each shoulder).

In a further instance of the disclosure, there is provided a shoulder stabilising kit comprising a pressure pad, and optionally one or more protruding members. In a further instance, the kit additionally comprises one or more retention members. In one instance, the pressure pad is configured to be removably attachable to the one or more retention members.

In a further aspect of the invention there is provided a garment comprising or adapted to retain one or more shoulder stabilising devices as hereinbefore defined. Examples of such garments include but are not limited to a shirt, T-shirt, vest, brassiere, upper body portion of sports clothing or leotard. Such garments can be designed with pockets which are configured to receive the shoulder stabilising device and which are located in precisely the right position to provide support to the glenohumeral joint. In one embodiment, the garment is a vest. The benefit of such a garment is that it will hold the shoulder stabilising device in position without the need for retention means and at the same time be conveniently worn underneath outer clothing.

In another aspect of the invention, there is provided a shoulder support comprising or adapted to retain one or more shoulder stabilising devices as hereinbefore defined. Examples of such shoulder supports include but are not limited to Bauerfeind® Omotrain® Shoulder Support. Such shoulder supports can be designed with pockets which are configured to receive the shoulder stabilising device and which are located in precisely the right position to provide support to the glenohumeral joint.

According to another aspect of the invention there is provided a use of the shoulder stabilising device as hereinbefore defined for applying a focal pressure to an anterior margin (e.g. the lesser tuberosity/tubercle) of a glenohumeral joint, without applying direct pressure to the humeral head, so as to cause posterior translation of the humeral head with the glenohumeral joint.

According to another instance of the disclosure there is provided a method of stabilising a shoulder in a human subject which comprises the step of applying a focal pressure to an anterior margin (e.g. the lesser tuberosity/tubercle) of a glenohumeral joint of the subject so as to cause posterior translation of the humeral head within the glenohumeral joint. In one instance, the focal pressure is applied by means of a shoulder stabilising device as hereinbefore defined.

In a further instance of the disclosure, there is provided a shoulder stabilising device as hereinbefore defined for use in the treatment or prevention of shoulder impingement pain in a human subject. The treatment or prevention comprises the step of applying a focal pressure to an anterior margin (e.g. the lesser tuberosity/tubercle) of a glenohumeral joint of the subject so as to cause posterior translation of the humeral head within the glenohumeral joint.

In a further instance of the disclosure, there is provided a shoulder stabilising device as hereinbefore defined for use in the treatment or prevention of a partial or full thickness rotator cuff muscle tear. The treatment or prevention comprises the step of applying a focal pressure to an anterior margin (e.g. the lesser tuberosity/tubercle) of a glenohumeral joint of the subject so as to cause posterior translation of the humeral head within the glenohumeral joint.

According to a further instance of the disclosure, there is provided a method of treating or preventing shoulder impingement pain by applying to a subject a shoulder stabilising device as hereinbefore defined. The treatment or prevention comprises the step of applying a focal pressure to an anterior margin (e.g. the lesser tuberosity/tubercle) of a glenohumeral joint of the subject so as to cause posterior translation of the humeral head within the glenohumeral joint.

According to another instance of the disclosure, there is provided a method of treating or preventing a partial or full thickness rotator cuff muscle tear by applying to a subject a shoulder stabilising device as hereinbefore defined. The treatment or prevention comprises the step of applying a focal pressure to an anterior margin (e.g. the lesser tuberosity/tubercle) of a glenohumeral joint of the subject so as to cause posterior translation of the humeral head within the glenohumeral joint.

The invention will now be described, by way of example only, with reference to the accompanying figures, in which:
**Figures 1-22** show various embodiments of the shoulder stabilising device;
**Figure 23** shows a photograph of one embodiment of the shoulder stabilising device worn by a subject;
Figure 24 A-R shows numerous other possible embodiments of the shoulder stabilising device comprising retention members;
**Figure 25** shows a garment comprising a pocket to secure a shoulder stabilising device into position; and
**Figure 26** shows a garment comprising a shoulder stabilising device that has an inflatable member.

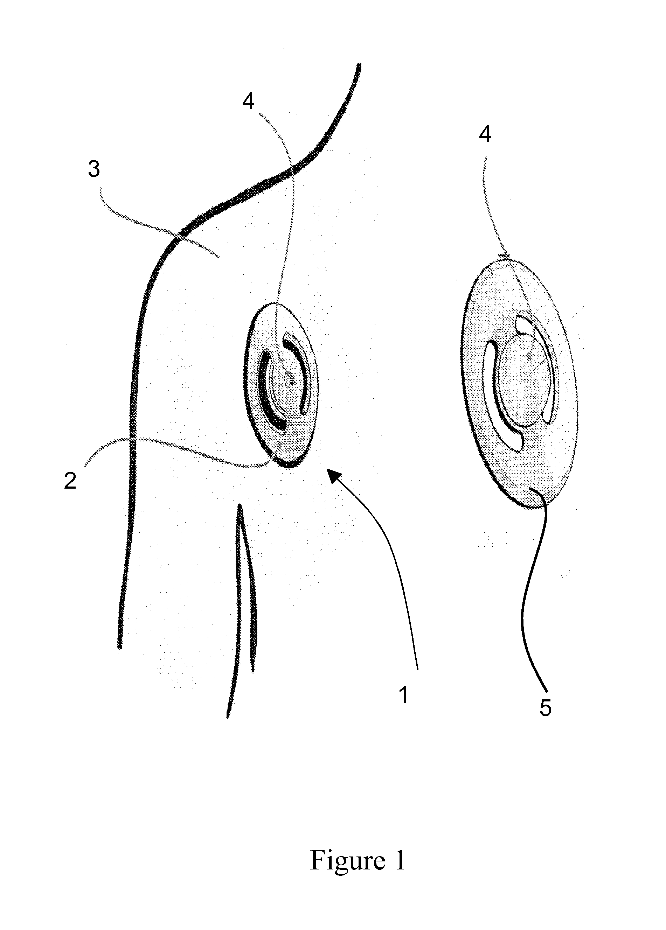

Referring first to Figure 1, a shoulder stabilising device is shown generally as 1. The main body of the device 1 forms the pressure pad 2 which lies flat on the skin of the subject 3. The protruding member 4 sits in the centre of the inner side of the pressure pad 5 and is urged away from the inner side of the pressure pad 5 by a spring (not visible). The protruding member 4 can vary in size and material corresponding to the needs of the subject.

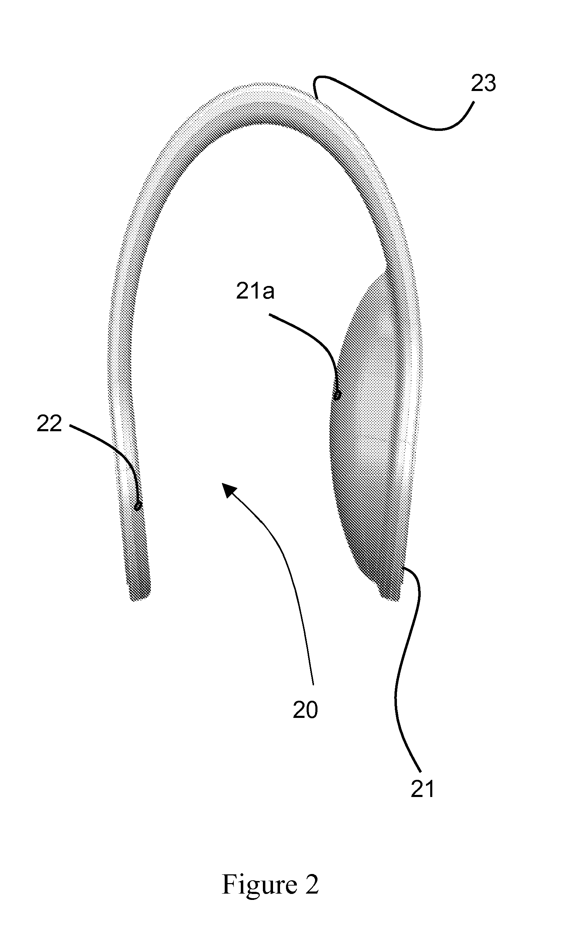

Figure 2 shows a shoulder stabilising device 20 comprising a pressure pad 21 and a support pad 22. The pressure pad 21 and the support pad 22 are connected by a joining portion 23. The pressure pad has a protruding member 21 a.

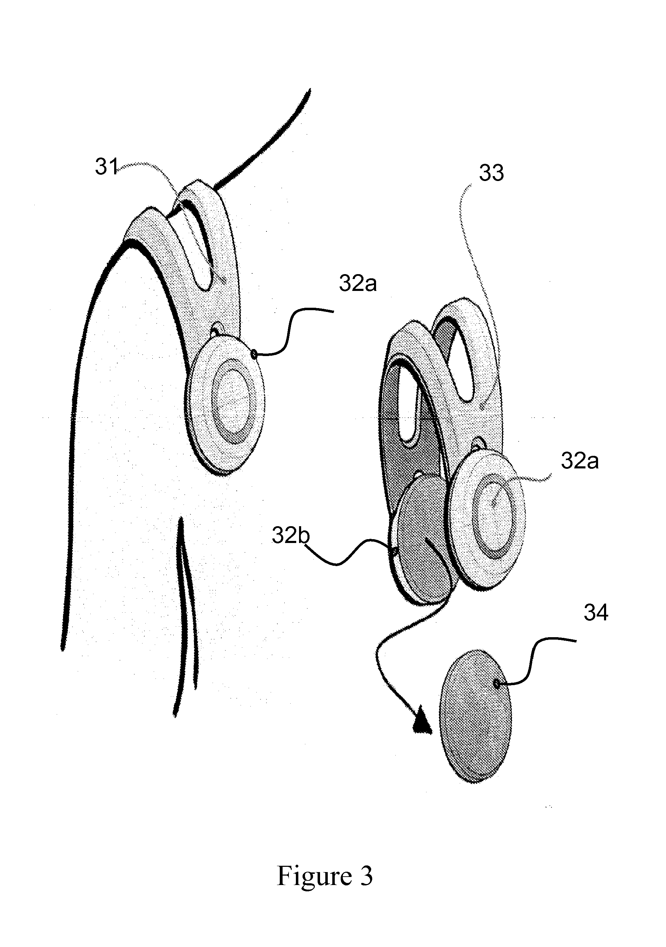

A shoulder stabilising device 31 comprising a pressure pad 32a and a support pad 32b is shown in Figure 3. The pressure pad 32a and the support pad 32b are connected by a joining portion 33, which branches into two separate sections and has a substantially flat cross-section making the device 31 comfortable to wear and unobtrusive. The surface of pressure pad 32a and the support pad 32b is coated with an adhesive material 34. In use, the shoulder stabilising device 31 rests on the surface of the shoulder with the branched joining portion 33 and the support pad 32b securing the position of the pressure pad 32a, and preventing unwanted lateral movement. The pressure pad 32a applies pressure to the anterior margin of the GH joint. The adhesive surface 34 of the pressure pad 32a and the support pad 32b allows them to stick to the subject's skin further securing the position of the shoulder stabilising device 31.

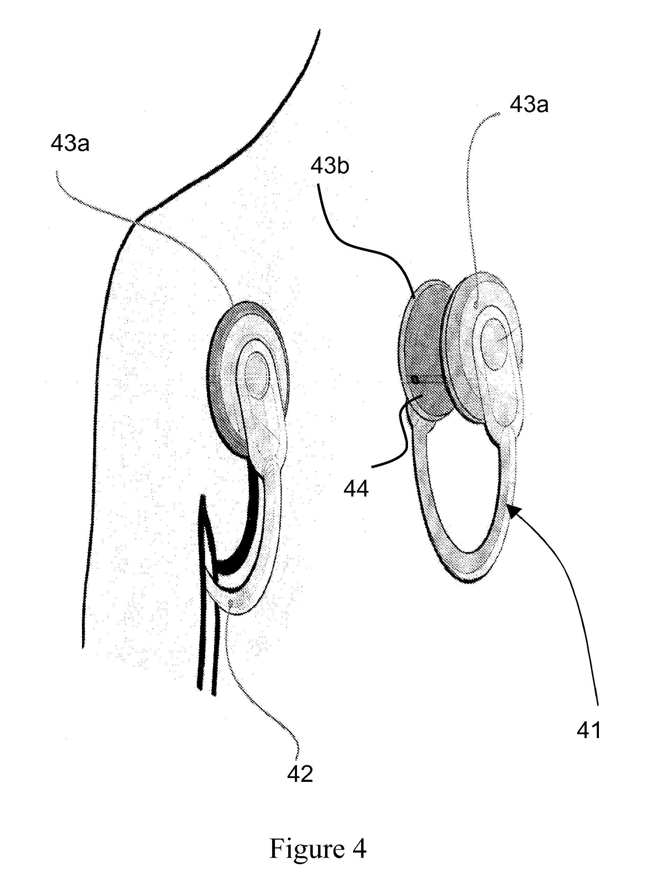

Figure 4 generally shows a shoulder stabilising device as 41, which comprises a joining portion 42 that forms a loop around the bottom of the shoulder. Both the pressure pad 43a and the support pad 43b have an adhesive coating 44. In use, the surface 44 of the pressure pad 43a and the shoulder pad 43b stick to the skin of the subject, thereby maintaining the shoulder stabilising device 41 in the correct position. The pressure pad 43a applies focal pressure to the anterior margin of the GH joint.

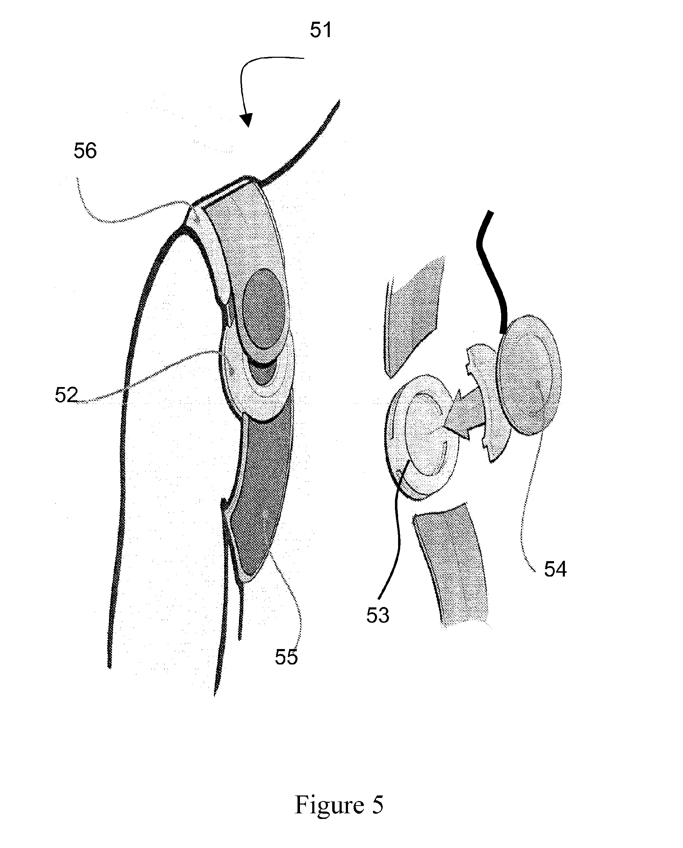

A further embodiment of the shoulder stabilising device is generally shown as 51 in Figure 5. The pressure pad 52 comprises a peripheral portion 53 configured to maintain contact with the shoulder of the subject and a central portion configured to accommodate the protruding member 54. In this embodiment the retention member is a support strap 55 with a buckle 56 for pressure adjustment. In use, the single protruding member 54 snaps into the peripheral portion 53 which forms part of the pressure pad 52. The amount and nature of pressure applied by the device 51 can be altered by exchanging the protruding member 54 with a protruding member having a different size and/or shape according to the needs of the subject.

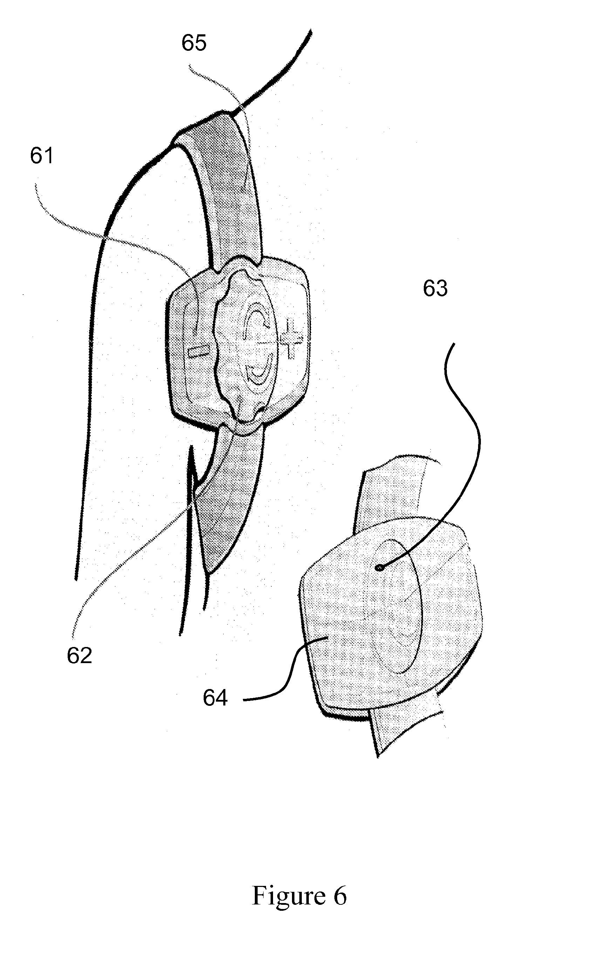

Figure 6 shows another embodiment of the shoulder stabilising device 61. In this embodiment, the shoulder stabilising device 61 comprises a screw adjustment 62 to vary the amount of pressure exerted on the anterior margin of the shoulder. A protruding member 63 is located on the inner surface of the pressure pad 64. The shoulder stabilising device 61 is held in position by means of a support strap 65. In use, turning the screw adjustment 62 in one direction will cause the support strap to tighten, thereby causing the protruding member 63 to move towards the anterior margin of the shoulder and increasing the pressure applied to the latter. Turning the screw 62 in the other direction will have the opposite effect.

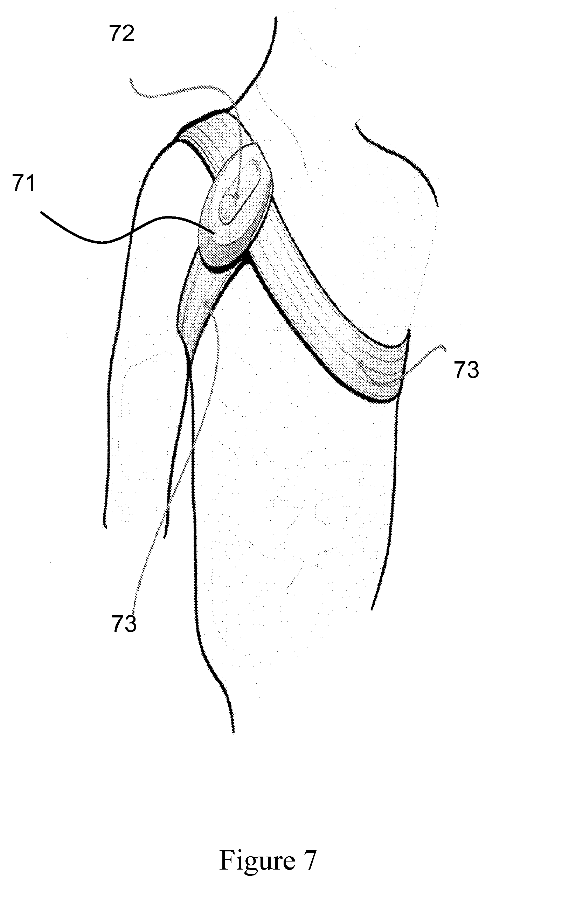

A further embodiment is shown in Figure 7, wherein the pressure applied by the shoulder stabilising device 71 is adjusted by a ratchet mechanism 72. In use, the degree of pressure applied by the pressure pad (not shown) is altered by moving the ratchet 72. A support harness 73 supports the pressure pad 71 on the subject in this figure.

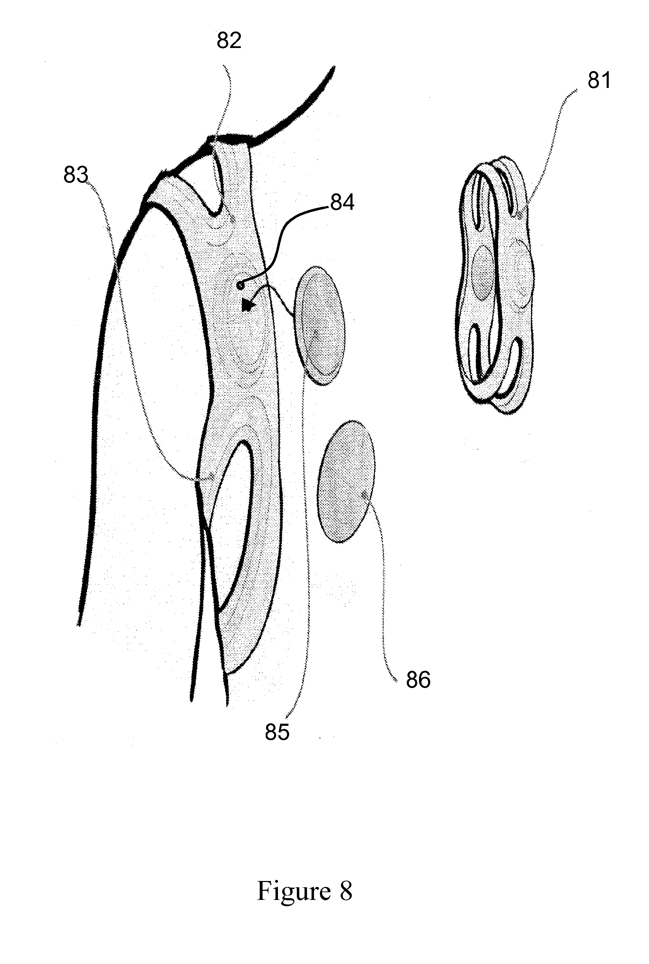

Figure 8 shows a shoulder stabilising device 81, which comprises an elastic support strap 82. The elastic support strap 82 is branched 83 and comprises a portion 84 for receiving a pressure pad 85 (indicated by the solid arrows in Figure 8). The inner side of the pressure pad 85 comprises a protruding member 86. In use, the pressure pad 85 is held in position by the elastic support strap 82. The branched nature of the support strap allows it to more effectively grip the surface of the subject's shoulder.

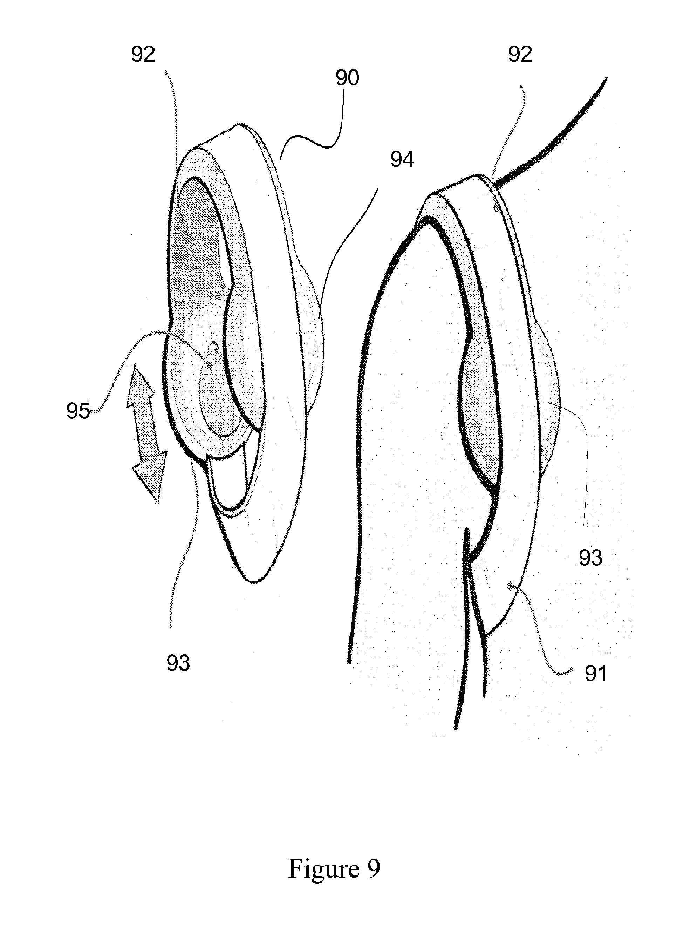

The shoulder stabilising device 90 shown in Figure 9 comprises a support strap 91 of a rigid, non-elastic material, a joining portion 92, a pressure pad 93 and a support pad 94. The protruding member 95 of this shoulder stabilising device 90 can be moved to different positions within the pressure pad 93 (as shown by arrow). When in use, the adjustable protruding member 95 enables pressure to be applied to the required part of the anterior margin (e.g. the lesser tuberosity/tubercle).

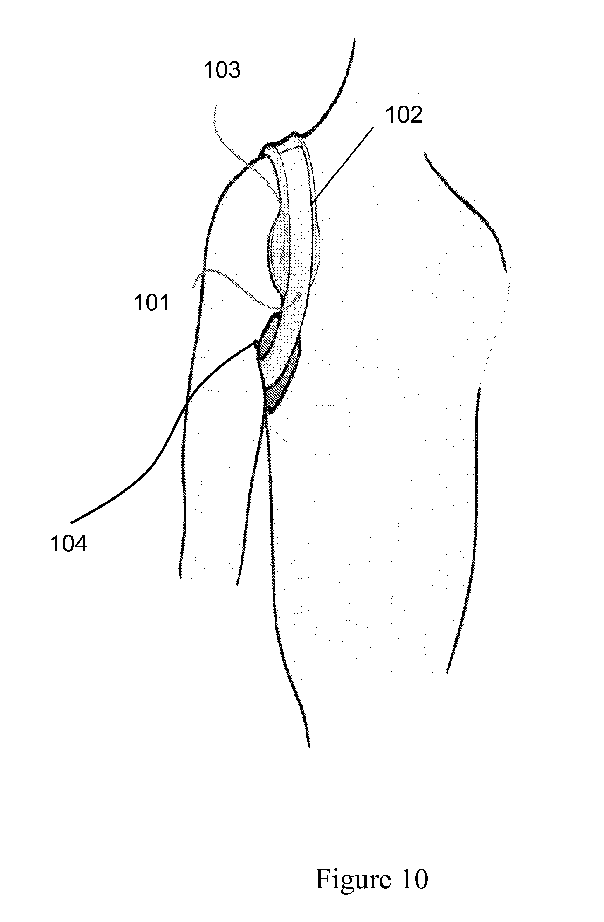

Figure 10 shows the same shoulder stabilising device as in Figure 9, comprising a support strap 101 of a rigid, non-elastic material, a joining portion 102, a pressure pad 103 and a support pad (not shown). This shoulder stabilising device is combined with an under arm support 104, which in use, will make wearing the shoulder strap more comfortable for the subject.

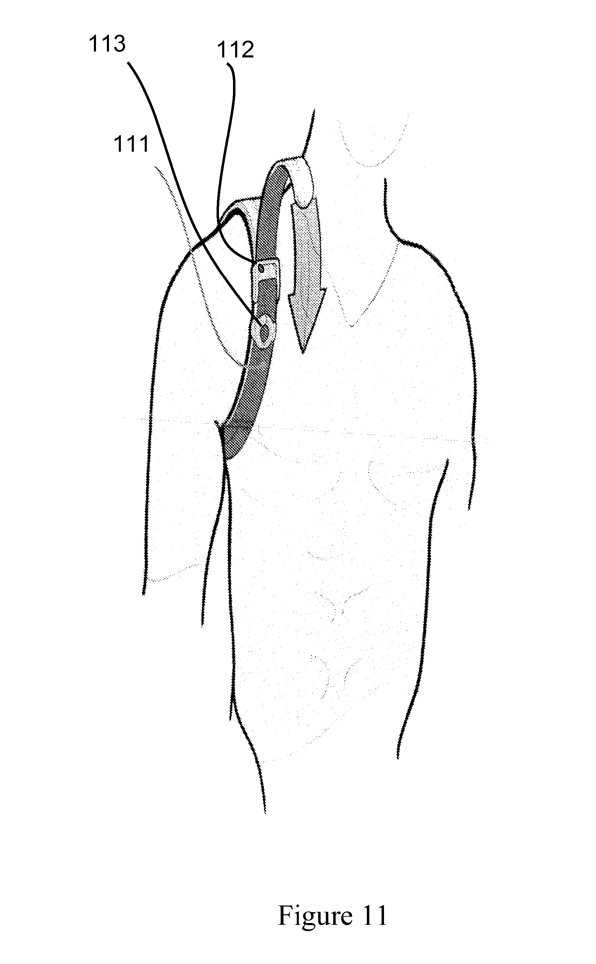

In Figure 11, a shoulder stabilising device comprising a support strap 111 with a buckle 112 and pressure pad 113 is shown. In use, the pressure pad 113 is placed in the correct position and the support strap 111 is tightened by being drawn through the buckle 112 and fastened to back of the remainder of the support strap 111 by, for example, a Velcro® fitting (as indicated by the arrow).

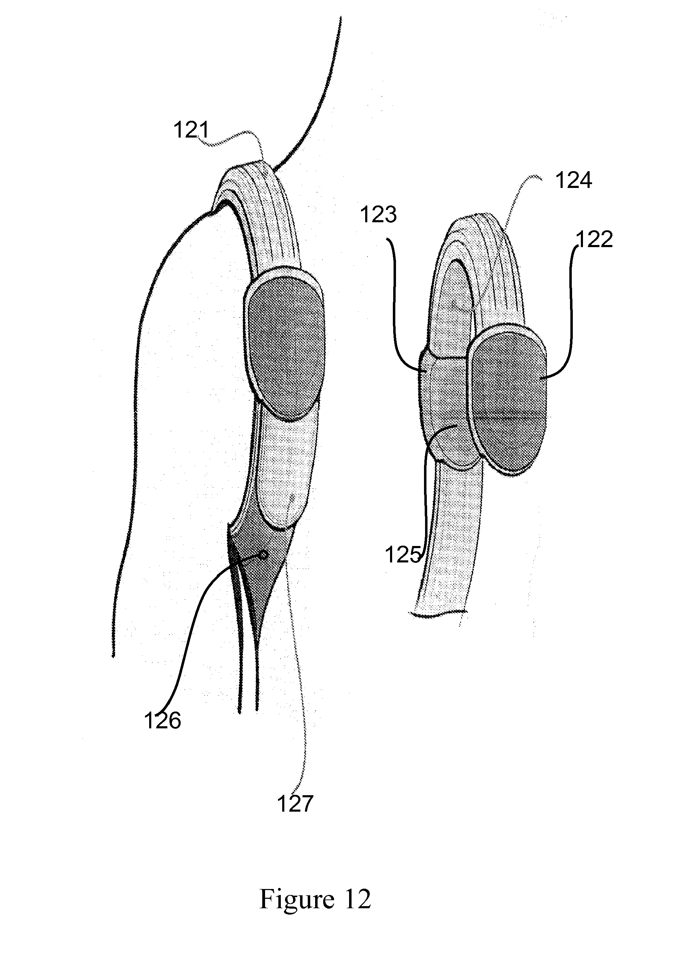

Figure 12 shows another embodiment of the shoulder stabilising device 121. The shoulder stabilising device 121 is equipped with a pressure pad 122 and a support pad 123 and a joining portion 124 with a padded inner surface. The surface 125 of the support pad 123 is flat and the surface of the pressure pad 122 contains a protruding member (not shown). A support strap 126 is attached to the support pad 123 and the pressure pad 122 and is secured into position with a Velcro® portion 127.

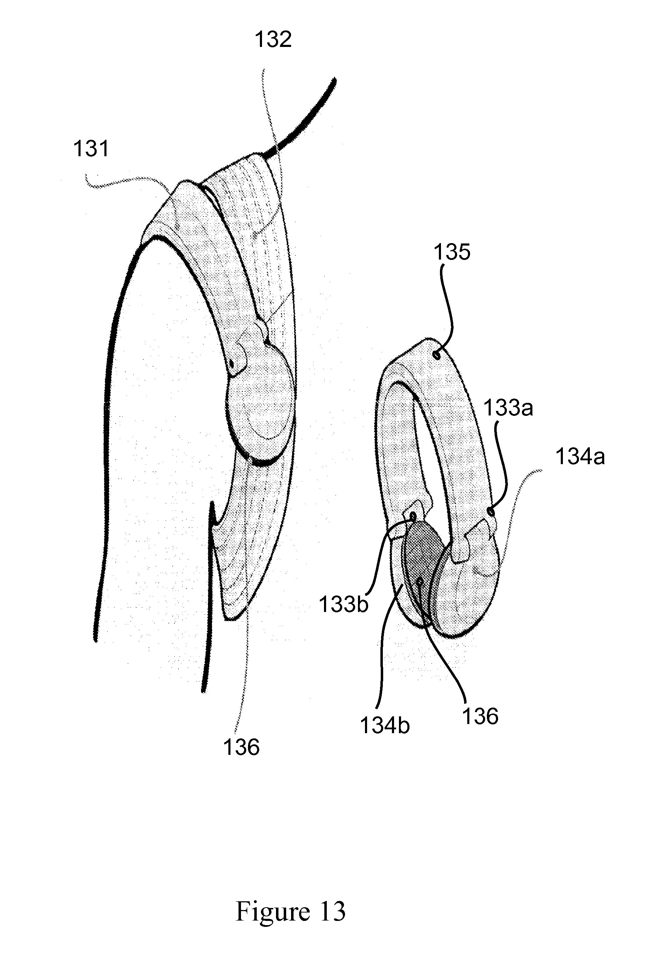

The shoulder stabilising device 131 shown in Figure 13 comprises a pressure pad 134a and a support pad 134b and a joining portion 135, which can be attached and detached to an elastic support strap 132. Hinges 133a and 133b ensure that the pressure pad 134a and the support pad 134b are articulated with respect to the joining portion 135. The surfaces of the pressure pad 134a and the support pad 134b are covered in Velcro® material 136. The pressure pad 134a is urged towards the anterior margin by springs (not shown) contained within the hinge 133a. The Velcro surfaces 136 allow the pressure pad 134a to be removed and reattached to the support strap 132 as required. The pressure pad 134a can be placed at any position on the support strap 132 in order to apply the required pressure to the anterior margin.

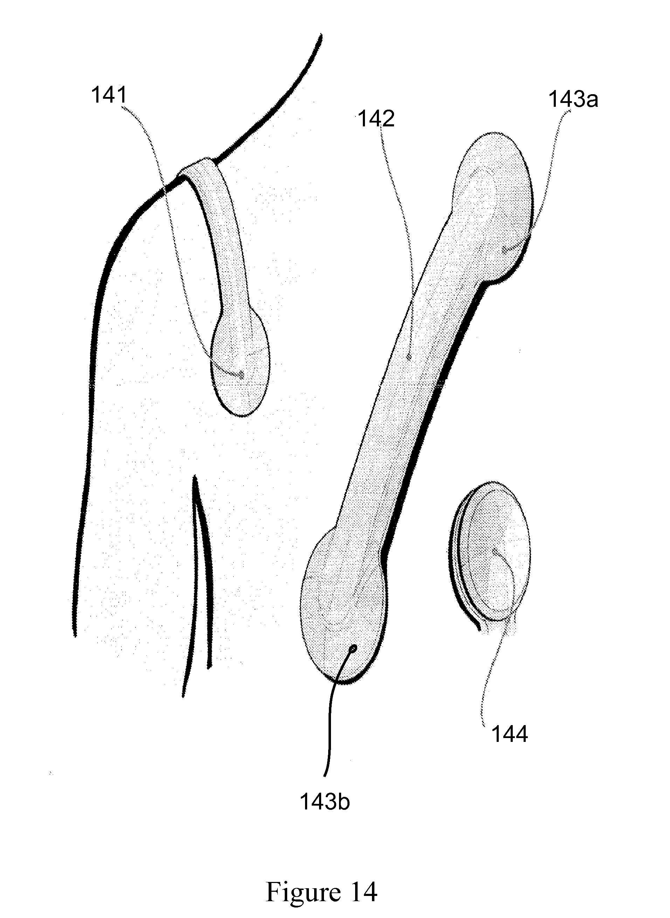

Figure 14 demonstrates an embodiment of the shoulder stabilising device 141 that is substantially flat. The shoulder stabilising device comprises a pressure pad 143a and a support pad 143b connected to each other by means of a joining portion 142. There is a protruding member 144 on the inner surface of the pressure pad 143a. In use, the joining portion 142 exerts no force on the pressure pad 143a. This shoulder stabilising device 141 can be combined with a retention member that urges the pressure pad 143a towards the anterior margin.

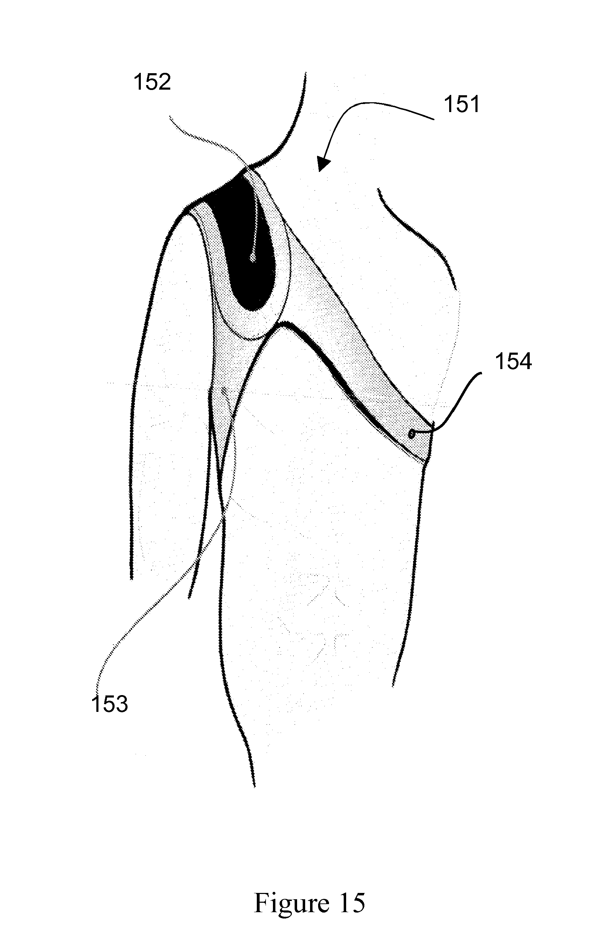

Such a retention member is shown generally as a compressive support harness 151 in Figure 15. The portion of the support harness 151 covering the shoulder 152 is constructed of a stiff flexible material and in use, urges the pressure pad (hidden beneath the support harness 151) against the anterior margin. Other sections of the support harness 151 run underneath the affected shoulder 153 and the opposite shoulder 154 to stabilise the shoulder stabilising device (not visible).

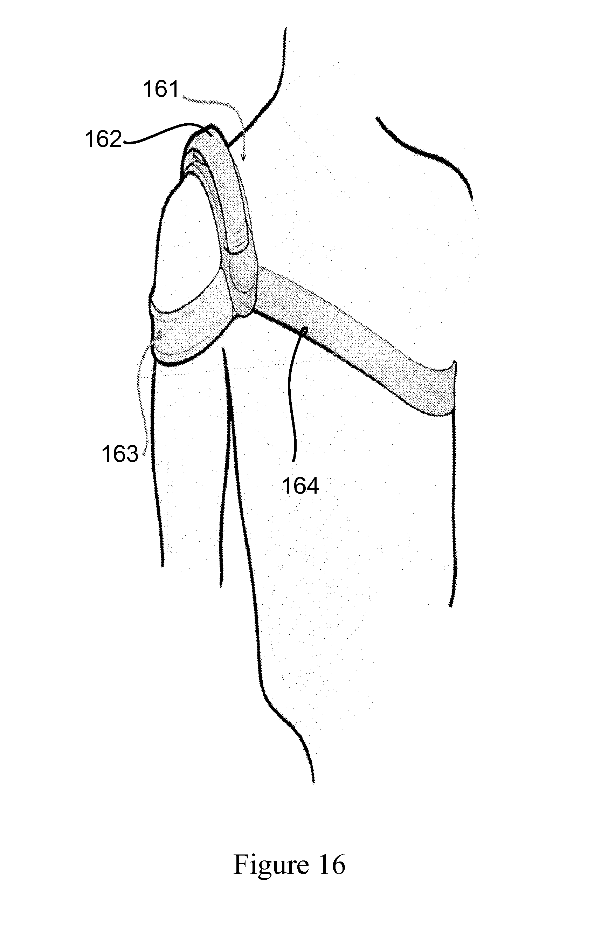

Another retention member is shown in Figure 16 as a support harness 161 with a shoulder strap 162, a portion horizontally encircling the affected shoulder 163 and a portion encircling the chest 164 of the subject. In use, the support harness 161 exerts downward force on the shoulder strap 162 and ensures the pressure pad (not shown) is held in the correct position.

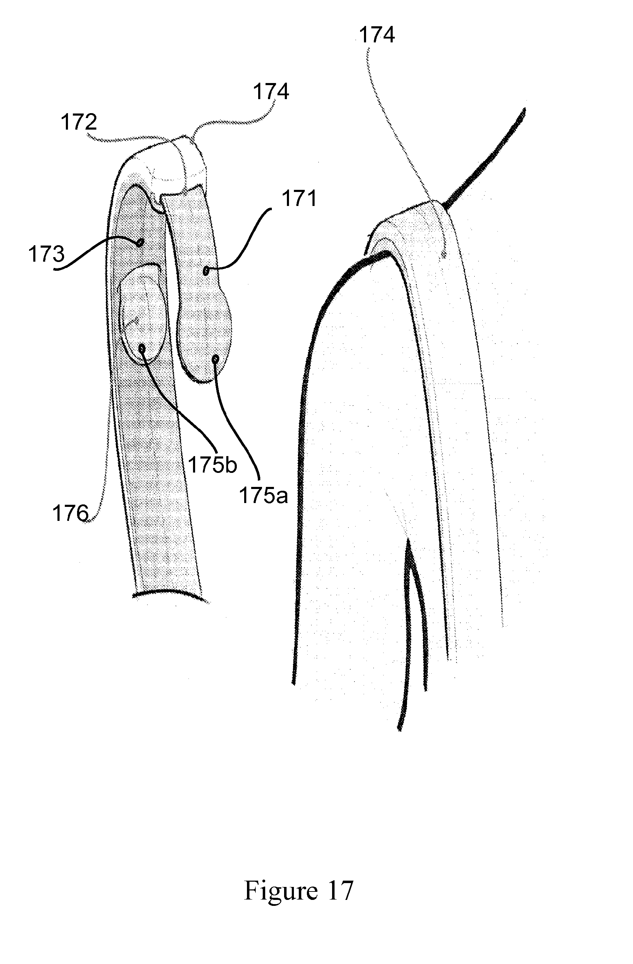

A further shoulder stabilising device 171 is shown in Figure 17. The device 171 comprises a joining portion 172, which connects a support pad 175a and a pressure pad 175b, the pressure pad 175b comprising a protruding member 176. The joining portion 172 has a substantially flat cross-section and can be introduced into a pocket 173 of a support harness 174. In use, the support harness 174 is designed to locate the pressure pad 175b in the correct position over the shoulder. The support harness 174 will be unobtrusive due to its slim design and is suitable when it is desirable to hide the presence of the support pad 175a, pressure pad 175b and the joining portion 172.

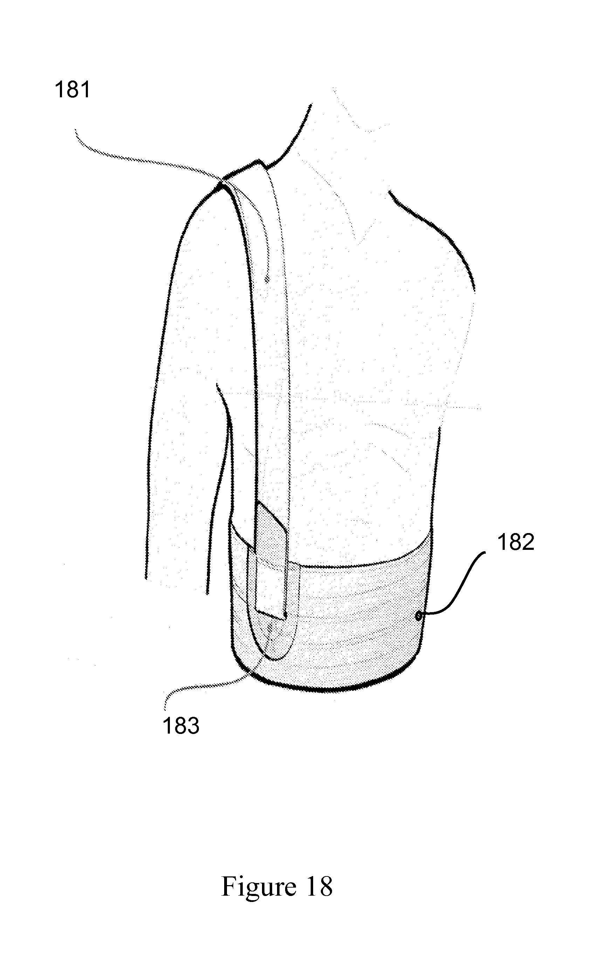

Figure 18 shows an embodiment of the shoulder stabilising device as described in Figure 17. The support harness comprises a support strap 181 which contains the pressure pad, support pad and joining portion (not visible) and a waistband 182 to which it is fastened with a buckle 183. In use, the support strap 181 covers the shoulder and descends to waist level along the front and back of the subject where it joins to the waistband 182. The waistband 182 ensures that downward force is applied to the shoulder strap 181 which maintains the shoulder stabilising device in the correct position.

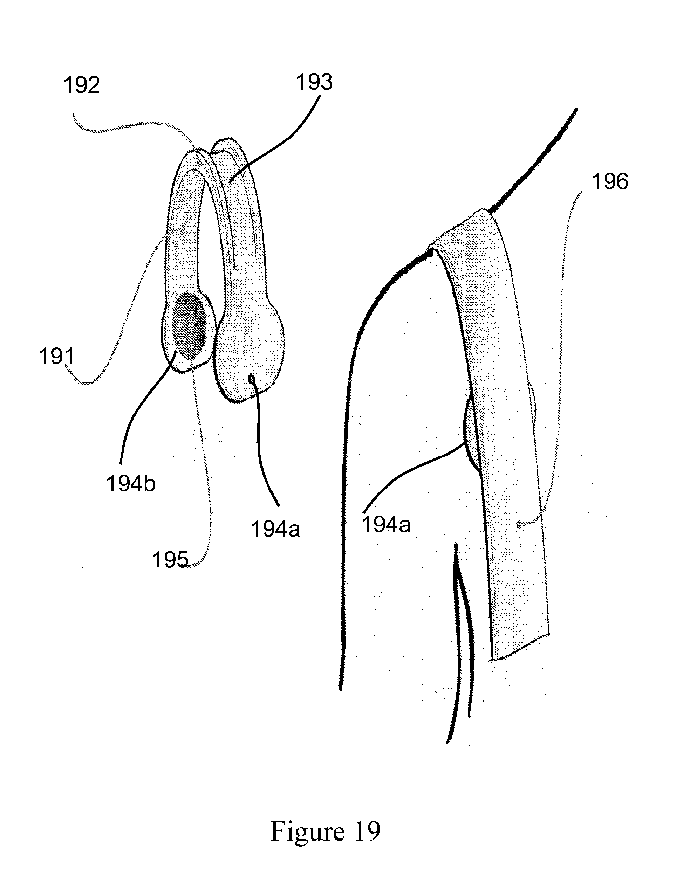

A slight variation of this embodiment is shown in Figure 19. The joining portion 192 of the shoulder stabilising device 191 comprises a groove 193. Support pad 194a and pressure pad 194b are located at the extremities of the joining portion, with the pressure pad 194b comprising a protruding member 195. In use, the joining portion 192 of this embodiment is not inserted into the support harness 196, but is instead covered by it. The groove 193 in the joining portion 192 ensures that the support harness 196 is properly guided and does not slip off the shoulder stabilising device 191. The slim appearance of this design is also suited for inconspicuous use of the shoulder stabilising device 191. In this embodiment a shoulder stabilising device 191 may be placed on each shoulder, supported in position by the support harness 196.

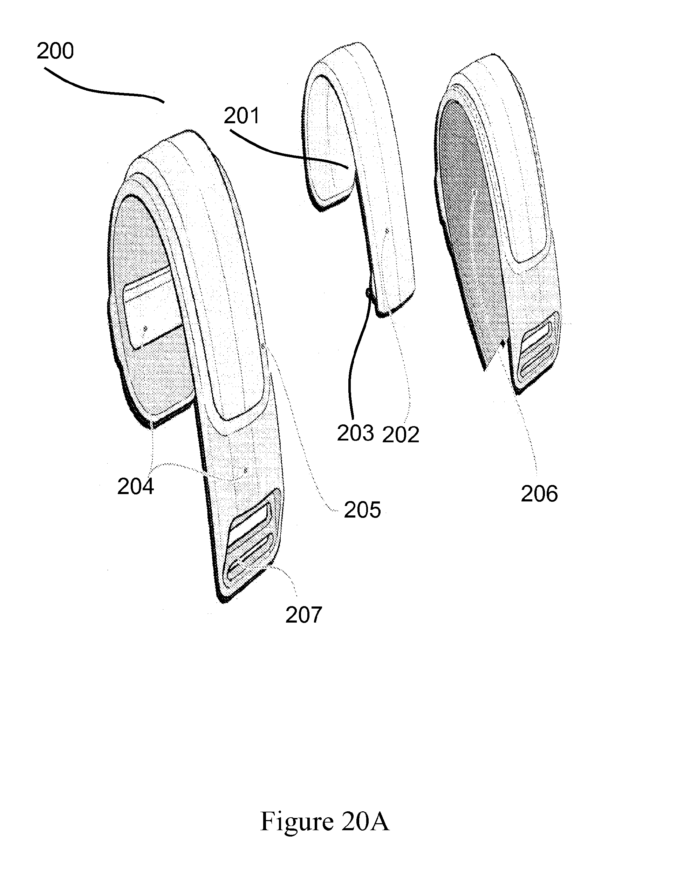

Figure 20A shows a further embodiment of a shoulder stabilising device 200. The shoulder stabilising device 200 comprises a support strap 201, having a pressure pad 202. The pressure pad 202 comprises one protruding member 203. The support strap 201 is inserted into the pockets 204 of a shoulder support strap 205 which comprises soft padding 206 on the inner surface. Slots 207 in the shoulder support strap 205 are designed to receive a support harness (not shown).

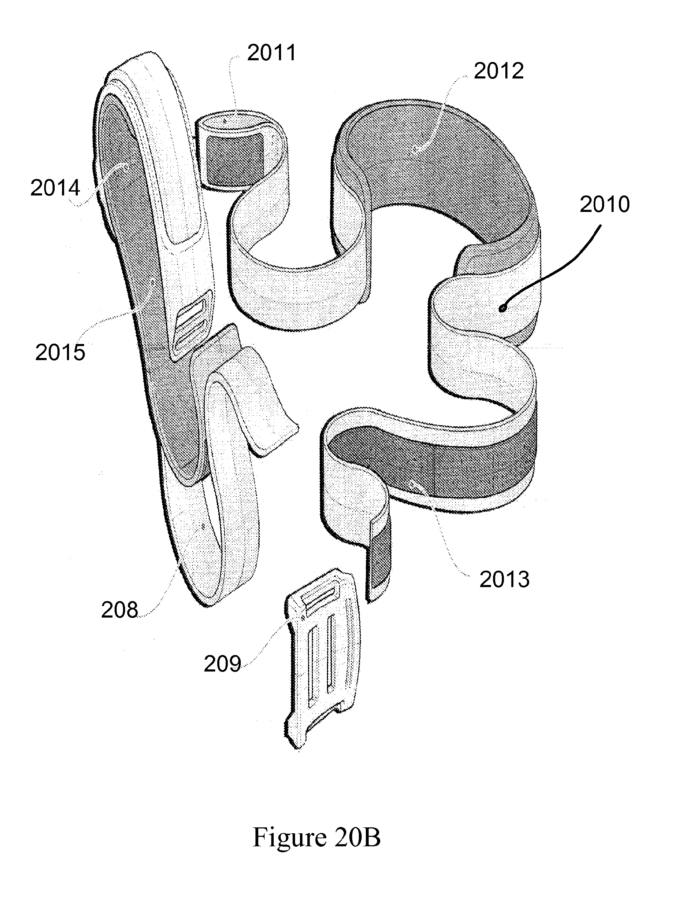

The complete shoulder stabilising device is shown in Figure 20B. The shoulder support strap 2014 with soft inner padding 2015 is shown, as well as, an adjustment strap 208, a buckle 209 and a support harness 2010 which comprises a loop 2011 for connection to the shoulder support strap 2014, a padded portion 2012 and a Velcro portion 2013 for folding over any excess length of harness.

In use, the shoulder support strap 2014 is applied to the shoulder and attached to the support harness 2010 by means of the buckle 209. The support harness 2010 serves to secure the shoulder support strap 2014 in the correct position. The padded sections of both the shoulder support strap 2015 and the padded portion 2012 improve comfort to the subject using the device.

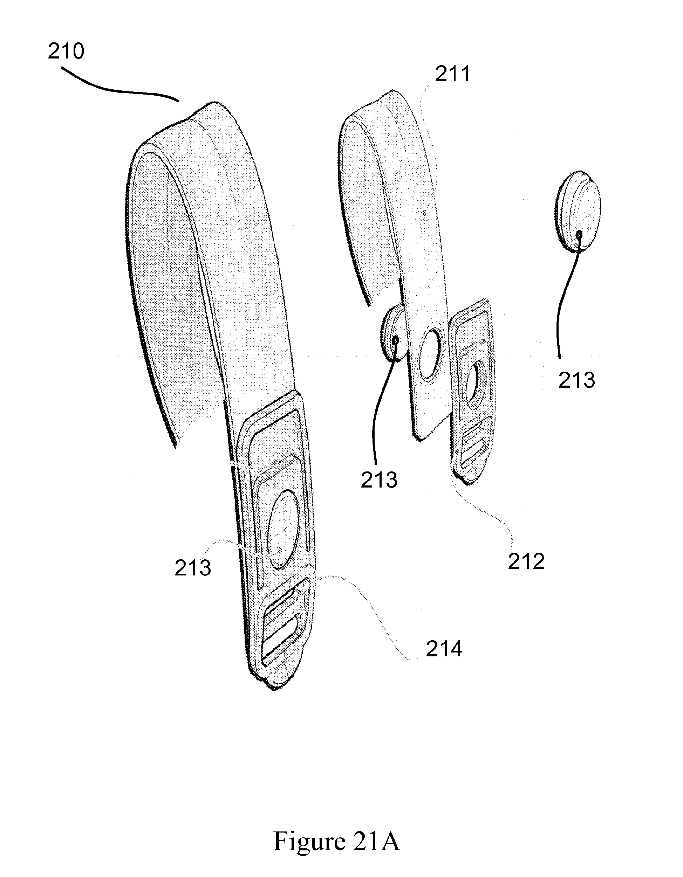

A similar embodiment is shown in Figure 21A. The shoulder stabilising device 210 comprises a shoulder support strap 211 and a pressure pad 212 with an interchangeable protruding member 213. Slots 214 in the shoulder support strap 211 allow attachment of the shoulder support strap 211 to a support harness (not shown).

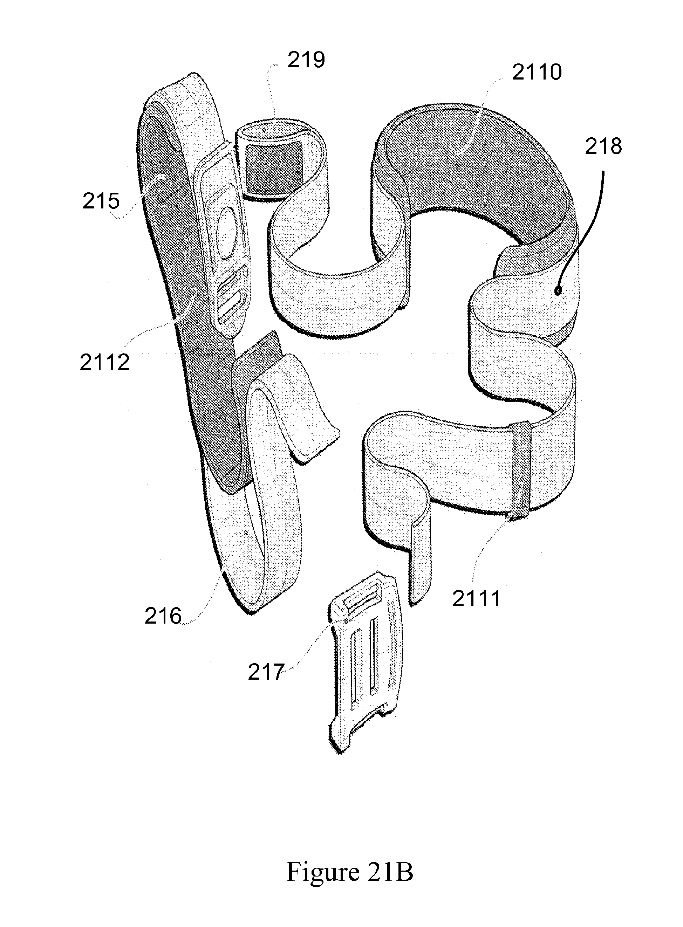

The complete shoulder stabilising device is shown in Figure 21 B. The shoulder support strap 2112 is shown as well as the soft inner padding 215, an adjustment strap 216, a buckle 217 and a support harness 218 which comprises a loop 219 for connection to the shoulder support strap 2112, a padded portion 2110 and a second loop 2111 for folding over any excess length of harness.

In use, the shoulder support strap 2112 is applied to the shoulder and attached to the support harness 218 by means of the buckle 217. The support harness 218 serves to secure the shoulder support strap 2112 in the correct position. The padded portion of the harness 2110 improves comfort to the subject using the device. The interchangeable protruding members 213 allow the pressure applied to the shoulder to be varied according to the subject's requirements.

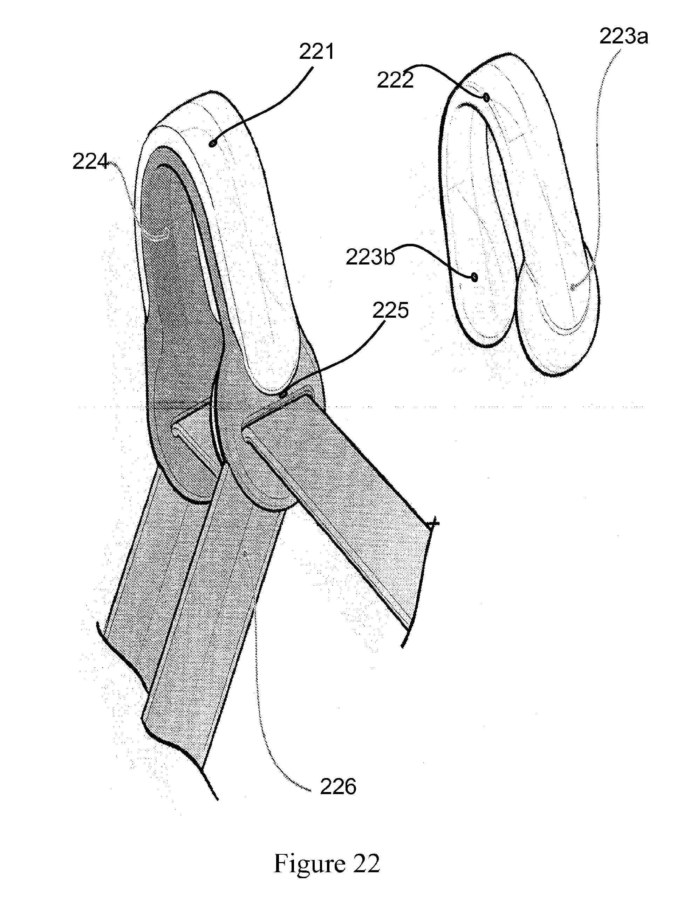

Yet another embodiment of the shoulder stabilising device is shown in Figure 22. The shoulder stabilising device 221 comprises a joining portion 222, a support pad 223b, a pressure pad 223a and a protruding member on the pressure pad (not visible). A shoulder support strap 224 comprises slots 225 for receiving a support harness 226. In use, the support harness 226 will exert downward force on the shoulder support strap 224 firmly holding it in position on the subject's shoulder.

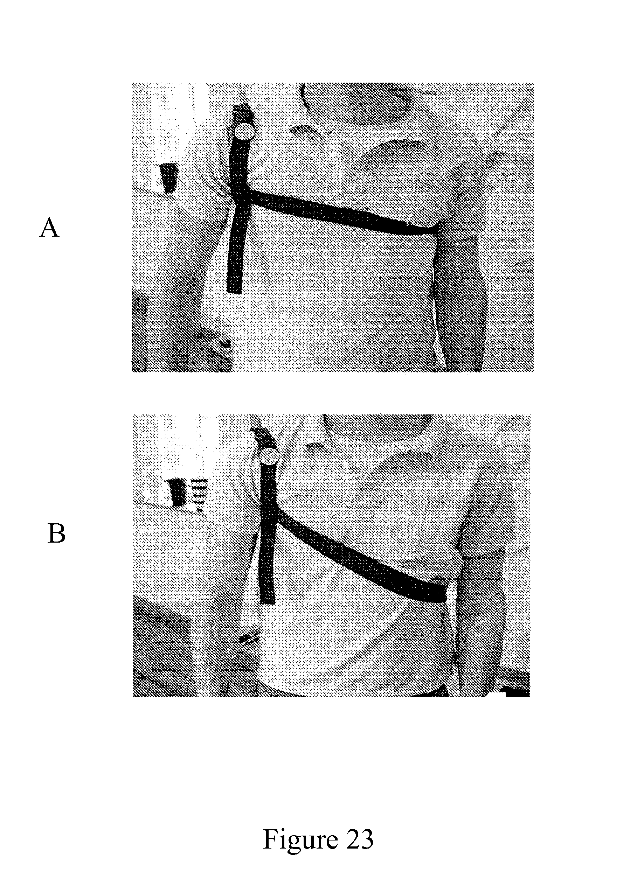

Photographs of a subject wearing a shoulder stabilising device are shown in Figures 23A and 23B. The supporting harness is worn high across the body in Figure 23A and low across the body in Figure 23B.

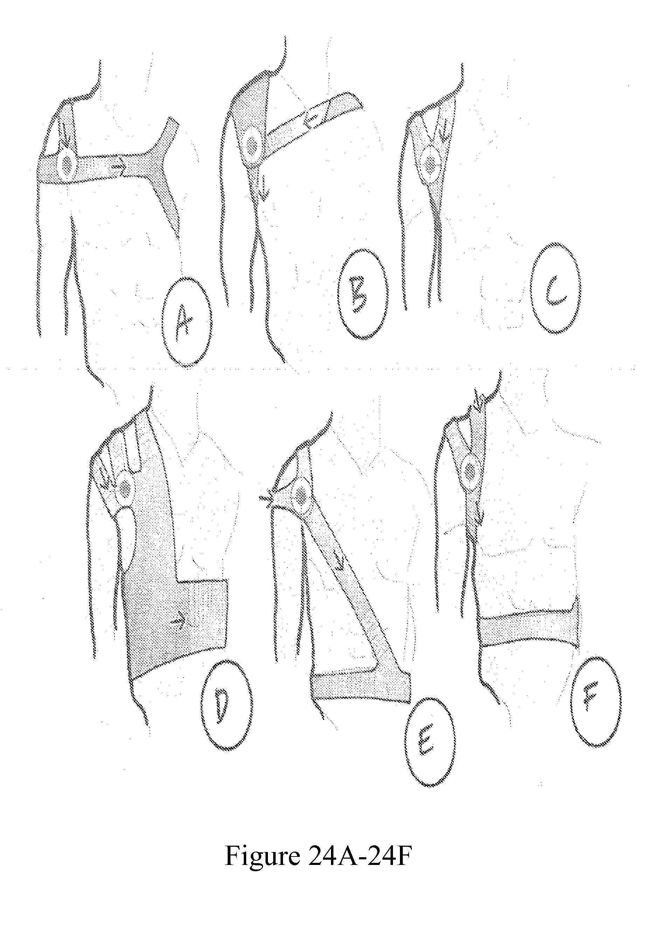

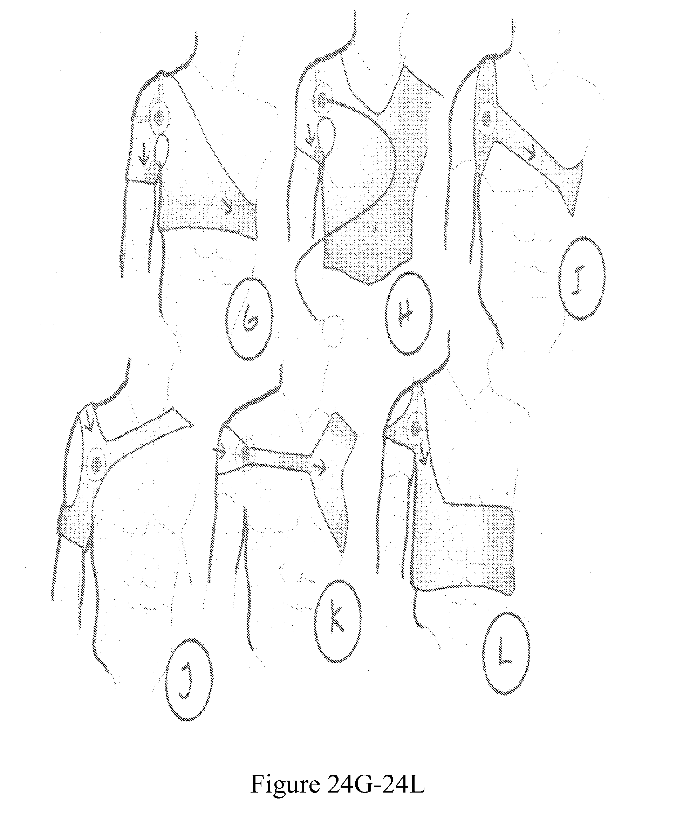

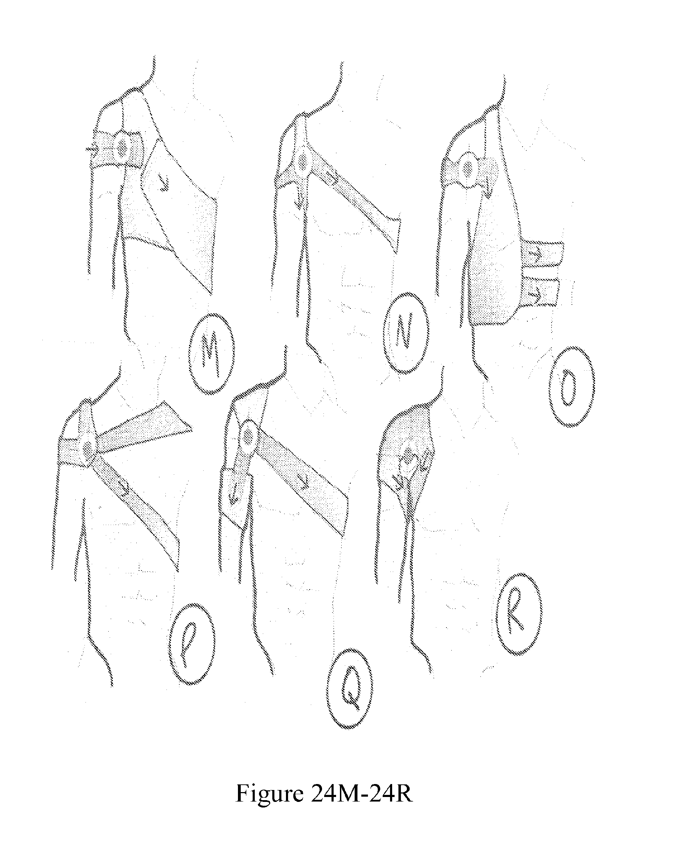

Figures 24A to 24R show many different possible embodiments of a shoulder stabilising device. The arrows indicate the direction of force applied by the strap and/or harness in each case.

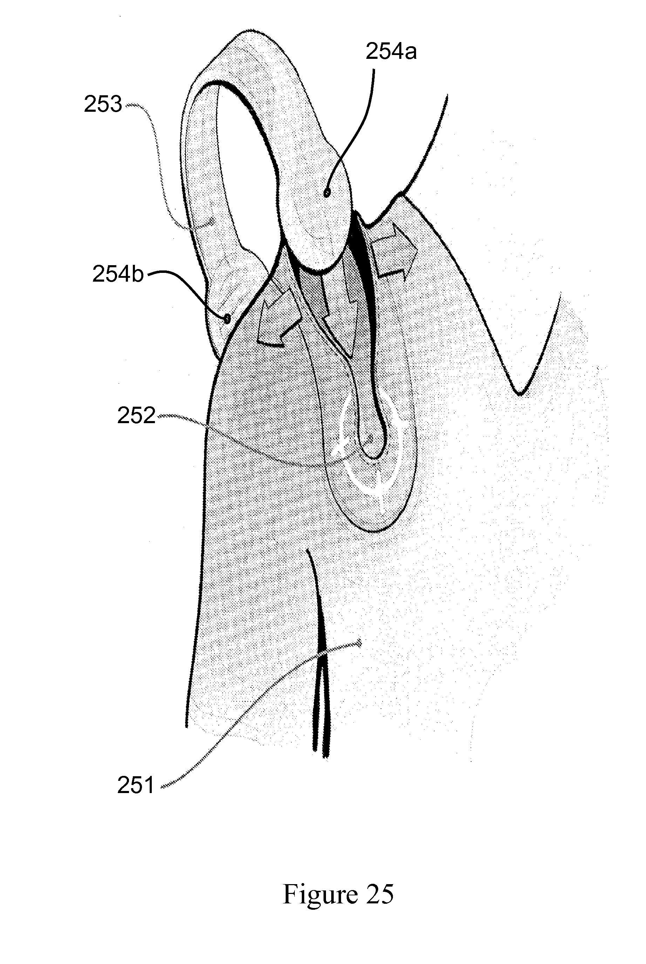

Figure 25 shows a garment incorporating a shoulder stabilising device 253. A tight-fitting vest 251 comprises a pocket 252 into which the shoulder stabilising device 253 comprising a pressure pad 254a and a support pad 254b can be inserted (as shown by the solid arrows in Figure 25). In use, the position of the pocket 252 within the garment 251 is carefully determined, to ensure that the pressure pad 254a and the support pad 254b are in the correct location. The tight-fitting nature of the vest 251 ensures that the device 253 remains in the desired position.

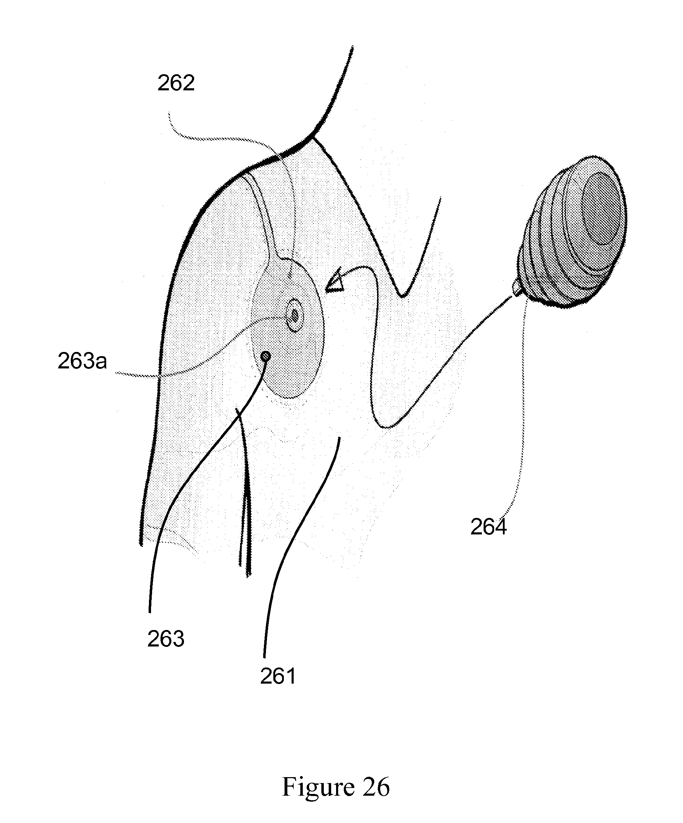

Another embodiment of the garment is shown in Figure 26. The vest 261 comprises a shoulder stabilising device 262 comprising a pressure pad (not shown) equipped with an inflation member 263, and an inflation valve 263a. In use, a hand pump 264 is applied to the inflation valve 263a (as indicated by the arrow) and actuated in order to inflate the inflation member 263. The greater the degree of inflation, the greater the pressure exerted on the shoulder. As with the previous embodiment, the position of the device 262 within the garment 261 is chosen with precision to ensure utility of the garment in realigning the glenohumeral joint.

## Claims

1. A shoulder stabilising device (1) comprising:
a pressure pad (2) configured to be positioned on an anterior margin of a glenohumeral joint of a human subject;
**characterised in that** the shoulder stabilising device (1) is configured, in use, to apply a focal pressure, via the pressure pad (2), to the anterior margin of the glenohumeral joint, without applying direct pressure to the humeral head, whereby said focal pressure causes posterior translation of the humeral head within the glenohumeral joint.

2. The device of Claim 1, wherein the shoulder stabilising device applies the focal pressure, via the pressure pad, to the lesser tuberosity/tubercle.

3. The device of Claim 1 or Claim 2, wherein the pressure pad comprises one or more protruding members.

4. The device of Claim 3, wherein the pressure pad comprises a single protruding member.

5. The device of any of Claims 1-4, wherein the shoulder stabilising device additionally comprises a pressure adjustor selected from a screw adjustment mechanism, a ratchet mechanism and one or more inflatable members.

6. The device of any preceding claim, wherein the device additionally comprises one or more retention members.

7. The device of Claim 6, wherein the one or more retention members are selected from the group consisting of a support strap, a support band, a support belt, a support leash and/or a support harness.

8. The device of Claim 7, wherein the retention member is a support pad.

9. The device of Claim 8, wherein the device additionally comprises a joining portion that connects the pressure pad to the support pad, and wherein the joining portion is configured to urge the pressure pad towards the anterior margin.

10. A garment comprising one or more shoulder stabilising devices of any of Claims 1 to 9.

11. A shoulder support comprising one or more shoulder stabilising devices of any of Claims 1 to 9.

## Patentansprüche

1. Schulterstabilisierungsvorrichtung (1), die Folgendes umfasst:
ein Druckkissen (2), das dafür konfiguriert ist, an einem vorderen Rand eines Schultergelenks eines menschlichen Patienten angeordnet zu werden;
**dadurch gekennzeichnet, dass** die Schulterstabilisierungsvorrichtung (1) dafür konfiguriert ist, bei Gebrauch über das Druckkissen (2) einen Fokaldruck auf den vorderen Rand des Schultergelenkes auszuüben, ohne direkten Druck auf den Oberarmkopf auszuüben, wodurch der genannte Fokaldruck eine posteriore Translation des Oberarmkopfes in dem Schultergelenk bewirkt.

2. Vorrichtung nach Anspruch 1, wobei die Schulterstabilisierungsvorrichtung den Fokaldruck über das Druckkissen auf die kleinere Tuberositas bzw. das kleinere Tuberkel ausübt.

3. Vorrichtung nach Anspruch 1 oder Anspruch 2, wobei das Druckkissen ein oder mehrere vorstehende Elemente umfasst.

4. Vorrichtung nach Anspruch 3, wobei das Druckkissen ein einzelnes vorstehendes Element umfasst.

5. Vorrichtung nach einem der Ansprüche 1 bis 4, wobei die Schulterstabilisierungsvorrichtung zusätzlich einen Druckregulierer umfasst, der aus einem Schraubenverstellmechanismus, einem Ratschenmechanismus und einem oder mehreren aufblasbaren Elementen ausgewählt wird.

6. Vorrichtung nach einem der vorhergehenden Ansprüche, wobei die Vorrichtung zusätzlich ein oder mehrere Rückhalteelemente umfasst.

7. Vorrichtung nach Anspruch 6, wobei das eine oder die mehreren Rückhalteelemente aus der Gruppe ausgewählt werden, die aus Folgendem besteht: einem Stützriemen, einem Stützband, einem Stützgürtel, einer Stützleine und/oder einem Stützgeschirr.

8. Vorrichtung nach Anspruch 7, wobei das Rückhalteelement ein Stützkissen ist.

9. Vorrichtung nach Anspruch 8, wobei die Vorrichtung zusätzlich einen Verbindungsabschnitt umfasst, der das Druckkissen mit dem Stützkissen verbindet und wobei der Verbindungsabschnitt so konfiguriert ist, dass er das Druckkissen in Richtung des vorderen Randes drückt.

10. Kleidungsstück, das eine oder mehrere Schulterstabilisierungsvorrichtungen nach einem der Ansprüche 1 bis 9 umfasst.

11. Schulterstütze, die eine oder mehrere Schulterstabilisierungsvorrichtungen nach einem der Ansprüche 1 bis 9 umfasst.

## Revendications

1. Dispositif de stabilisation d'épaule (1) comprenant :
un tampon de pression (2) configuré pour être positionné sur une marge antérieure d'une articulation gléno-humérale d'une personne ;
**caractérisé en ce que** le dispositif de stabilisation d'épaule (1) est configuré, en service, pour appliquer une pression focale, par l'intermédiaire du tampon de pression (2), sur la marge antérieure de l'articulation gléno-humérale, sans appliquer de pression directe sur la tête humérale, moyennant quoi la pression focale entraîne une translation postérieure de la tête humérale au sein de l'articulation gléno-humérale.

2. Dispositif selon la revendication 1, le dispositif de stabilisation d'épaule appliquant la pression focale, par l'intermédiaire du tampon de pression, à la petite/au petit tubérosité/tubercule.

3. Dispositif selon la revendication 1 ou la revendication 2, dans lequel le tampon de pression comprend un ou plusieurs éléments saillants.

4. Dispositif selon la revendication 3, dans lequel le tampon de pression comprend un seul élément saillant.

5. Dispositif selon l'une quelconque des revendications 1 à 4, le dispositif de stabilisation d'épaule comprenant en outre un ajusteur de pression sélectionné parmi un mécanisme d'ajustement à vis, un mécanisme à crans et un ou plusieurs éléments gonflables.

6. Dispositif selon l'une quelconque des revendications précédentes, le dispositif comprenant de plus un ou plusieurs éléments de retenue.

7. Dispositif selon la revendication 6, dans lequel les un ou plusieurs éléments de retenue sont sélectionnés dans le groupe comprenant en une sangle de support, une bande de support, une ceinture de support, un cordon de support et/ou un harnais de support.

8. Dispositif selon la revendication 7, dans lequel l'élément de retenue est un tampon de support.

9. Dispositif selon la revendication 8, le dispositif comprenant en outre une partie de raccordement qui raccorde le tampon de pression au tampon de support, et dans lequel la partie de raccordement est configurée pour pousser le tampon de pression vers la marge antérieure.

10. Vêtement comprenant un ou plusieurs dispositifs de stabilisation d'épaule selon l'une quelconque des revendications 1 à 9.

11. Support d'épaule comprenant un ou plusieurs dispositifs de stabilisation d'épaule selon l'une quelconque des revendications 1 à 9.
